(19) Europäisches Patentamt
European Patent Office
Office européen des brevets

(11) Publication number: **0 452 004 A2**

(12) **EUROPEAN PATENT APPLICATION**

(21) Application number: **91302773.6**

(22) Date of filing: **28.03.91**

(51) Int. Cl.[5]: **C10M 149/22,** C10M 145/26, C10M 133/52, C10L 1/18, C10L 1/22, C08G 73/02, C07C 217/00

(30) Priority: **29.03.90 GB 9007048**

(43) Date of publication of application: **16.10.91 Bulletin 91/42**

(84) Designated Contracting States: **BE DE DK ES FR GB IT NL SE**

(71) Applicant: **BP Chemicals Limited**
**Belgrave House 76 Buckingham Palace Road**
**London, SW1W 0SU (GB)**

(72) Inventor: **Cook, Stephen James, BP Chemicals Limited**
**Salt End**
**Hull HU12 8DS (GB)**
Inventor: **Dobson, Ian Daivd, BP Chemicals Limited**
**Salt End**
**Hull HU12 8DS (GB)**
Inventor: **Cracknell, Robert Brian, BP Chemicals Limited**
**Salt End**
**Hull HU12 8DS (GB)**

(74) Representative: **Wilson, Michael John et al**
**BP International Limited, Patents & Agreements Division, Chertsey Road**
**Sunbury-on-Thames, Middlesex TW16 7LN (GB)**

(54) **POLYALKOXYAMINES, THEIR PREPARATION, DISPERSANT ADDITIVES PREPARED THEREFROM AND USE OF THE ADDITIVES IN LUBRICATING OILS AND FUELS.**

(57) Disclosed are novel polyalkoxyamines having the formula :-

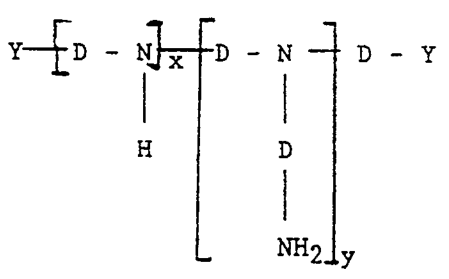

(I)

wherein (x+y) has a value in the range from 1 to 19 and y is either zero or an integer;

D is $-[CHR-CHR^1-O]_n-CHR^1-CHR-$ (II)

wherein either $R=R^1=H$ or $R=C_1$ to $C_4$ alkyl and $R^1=H$ and n has a value in the range from 1 to 10; and Y is independently either -OH or $-NHR^2$ wherein $R^2$ is either H or alkyl.

Polyalkoxyamines having the formula (I) are prepared by the deaminative polymerisation of diamines, which can be prepared by the catalysed reaction of a glycol and ammonia in the presence of hydrogen. Reaction of a hydrocarbyl-substituted succinic acylating agent with the polyalkoxyamine of formula (I) produces a dispersant additive suitable for use in lubricating oil and fuel compositions.

EP 0 452 004 A2

The present invention relates to polyalkoxyamines, processes for their preparation, dispersant additives prepared therefrom and to the use of the additives in lubricating oils compositions and fuels compositions.

A well known class of dispersant for use in lubricating oil compositions is that formed by the reaction of a hydrocarbyl-substituted succinic acylating agent, for example a polyisobutene succinic anhydride, and an amine, particularly a polyamine and more particularly a polyalkylene polyamine, for example triethylene tetramine (TETA) or tetraethylene pentamine (TEPA). For a review of the prior art on the production of such dispersants reference may be made to GB-A-1565627 which itself covers a lubricating composition comprising a major amount of oil of lubricating viscosity and a minor amount of one or more carboxylic derivatives produced by reacting at least one substituted succinic acylating agent with a reactant selected from (a) an amine having within its structure at least one H-N= group, (b) an alcohol, (c) a reactive metal or reactive metal compound, and (d) a combination of two or more of any of (a) to (c), the components (d) being reacted with said one or more substituted succinic acylating agents simultaneously or sequentially in any order, wherein said substituted succinic acylating agent(s) consist of substituent groups and succinic groups wherein the substituent groups are derived from polyalkene, said polyalkene having an $M_n$ value of 1300 to 5000 and a $M_w/M_n$ value of 1.5 to 4, said acylating agent(s) having within their structure an average of at least 1.3 succinic groups for each equivalent weight (as hereinbefore defined) of substituent groups.

Although polyamines, such as TETA and TEPA impart good dispersancy properties to, for example, polyisobutenesuccinimide dispersants formed from them, they also contribute to oil seal swelling problems in the engine in which lubricating oils containing them are employed. We have found that the problem of oil seal swelling can be reduced whilst retaining sufficient dispersancy by the use, in the preparation of dispersants from a hydrocarbyl substituted succinic acylating agent and a polyamine, of a polyamine containing both nitrogen and oxygen.

Accordingly, the present invention provides a polyalkoxyamine of the formula :-

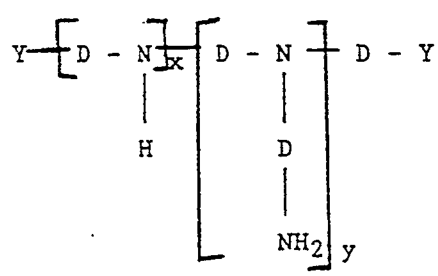

(I)

wherein (x+y) has a value in the range from 1 to 19 and y is either zero or an integer;

D is $\{CHR-CHR^1-O\}_nCHR^1-CHR-$ (II)

wherein in the formula (II) either $R=R^1=H$ or R= $C_1$ to $C_4$ alkyl and $R^1$= H and n has a value in the range from 1 to 10; and

Y is independently either -OH or $-NHR^2$ wherein $R^2$ is either H or alkyl.

With reference to the formula (I), (x+y) suitably has a value from 2 to 18, preferably less than 10, more preferably (x+y) is less than 5, and y is preferably either one or zero. Y is preferably $-NH_2$. With reference to the formula (II), n preferably has the value either two or three. R in the formula (II) may be methyl, ethyl, propyl or butyl, preferably methyl. Preferably $R=R_1=H$.

A preferred polyalkoxamamine has the formula (I) wherein:-

Y = $-NH_2$;

D = $\{CH_2-CH_2-O\}_nCH_2-CH_2-$ wherein n = either 2 or 3, preferably 2;

(x + y) has a value of either two or three and y is either zero or one.

Examples of polyoxyamines having the formula (I) include:-

$H_2N[CH_2CH_2OCH_2CH_2OCH_2CH_2NH]_4CH_2CH_2OCH_2CH_2OCH_2CH_2NH_2$ (III)

$H_2N[CH_2CH_2OCH_2CH_2OCH_2CH_2NH]_2CH_2CH_2OCH_2CH_2OCH_2CH_2NH_2$ (IV)

$H_2N[CH_2CH_2OCH_2CH_2NH]_2CH_2CH_2OCH_2CH_2NH_2$ (V)

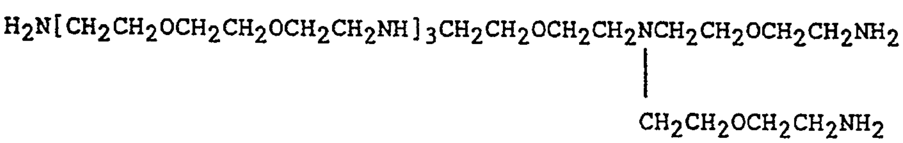

$H_2N[CH(CH_3)CH_2OCH(CH_3)CH_2NH]_2CH(CH_3)CH_2OCH(CH_3)CH_2NH_2$ (VII)

$H_2N[CH_2CH_2OCH_2CH_2NH]_2[CH(CH_3)CH_2OCH(CH_3)CH_2NH]CH_2CH_2OCH_2CH_2NH_2$ (VIII)

The invention also provides mixtures of two or more polyalkoxyamines having the formula (I), or mixtures thereof.

In another aspect the present invention provides processes for the production of polyalkoxyamines having the formula (I).

A first process for the production of a polyalkoxyamine having the formula (I), or a mixture thereof, comprises reacting at elevated temperature and in the presence of a transition metal catalyst a diamine having the formula:

$R^2HN[CHR-CHR^1-O]_nCHR^1-CHR-NHR^2$ (IX)

wherein in the formula (IX), R, $R^1$, $R^2$ and n have the same meaning as in the formulae (I) and (II).

$R^2$ in the formula (IX) is preferably hydrogen.

An example of a suitable diamine having the formula (IX) is triethyleneglycoldiamine. Compounds having the formula (IX) are commercially available and may be used without further purification.

In the process for producing a polyalkoxyamine having the formula (I) there is used a transition metal catalyst. Typically, as the transition metal either iron, cobalt or nickel may be employed. A suitable transition metal is nickel, which may be employed for example in the form of Raney nickel. Alternatively, the nickel may be supported on a suitable inert support, for example alumina.

The elevated temperature may suitably be in the range from 120 to 230°C, typically in the range from 140 to 210°C, preferably in the range from 170 to 210°C.

It is very much preferred to operate the process in an open system, that is a system in which evolved gases are allowed to vent. The process of the invention being a deaminative polymerisation, it is highly desirable that the ammonia evolved does not remain in contact with the reactants.

A second process for the production of a polyalkoxyamine having the formula (I) comprises in a first step reacting ammonia at elevated temperature and in the presence of a transition metal catalyst and hydrogen with a glycol having the formula:-

$HO[CHR-CHR^1-O]_nCHR^1-CHR-OH$ (X)

wherein in the formula (X) R,$R^1$ and n have the meanings assigned to them in respect of the formulae (I) and (II) to produce a diamine having the formula (IX) and in a second step reacting at elevated temperature and in the presence of a transition metal catalyst the diamine having the formula (IX).

Suitable transition metals for use in the first step include iron, cobalt and nickel. A preferred transition metal is nickel, which may be employed, for example, in the form of Raney nickel. Alternatively, the nickel may be supported on a suitable inert support.

Commercially available ammonia may be used in the first step with or without further purification. Commercially available hydrogen may also be employed, with or without purification.

Glycols having the formula (X) are commercially available and may be used in the first step without further purification. A suitable glycol of the formula (X) is triethyleneglycol.

In the first step the'elevated temperature may suitably be in the range from 120 to 250°C, preferably from 160 to 210°C.

As regards the second step the deaminative polymerisation of the diamine of the formula (IX) may suitably be accomplished in the manner as hereinbefore described.

Although the first and second steps may be operated independently in separate reactors, it is preferred to operate the first and second steps in the same reactor without isolation or purification of the diamine of the formula (IX).

In another aspect the invention provides a dispersant additive suitable for use in lubricating oil compositions which additive is obtainable by reacting at elevated temperature a polyalkoxyamine having the formula (I), or a mixture thereof, with a hydrocarbyl-substituted succinic acylating agent.

Suitable hydrocarbyl-substituted succinic acylating agents are described in the aforesaid GB-A-1565627. A preferred acylating agent has the formula:-

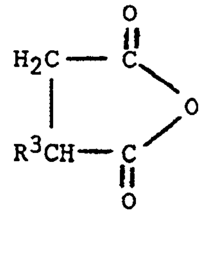

(XI)

wherein $R^3$ is an alkyl or alkenyl group derived from a polyolefin, preferably a polyisobutene.

The substituent $R^3$ in the formula (XI) may suitably have a number average molecular weight in the range from about 500 to about 5000, for example from about 500 to about 3000. Preferably the substituent $R^3$ has a number average molecular weight in the range from about 700 to about 2500, for example from about 1000 to about 2000. It is possible also to use a mixture of succinic acylating agents wherein the substituent $R^3$ is derived from polyolefins of differing number average molecular weight. $R^3$ may also be one or more succinic anhydride moieties, in which case the compound of formula (XI) would be a di- or poly- anhydride.

Generally, the hereinbefore described processes for producing polyalkoxyamines will produce a mixture of polyalkoxyamines of different molecular weights. Such mixtures are useful in the present invention without separation into their individual components.

A suitable polyalkoxyamine mixture for use in preparing the dispersant additives of the present invention is one obtainable by the deaminative polymerisation of triethyleneglycoldiamine and containing a polyalkoxyamine of the formula (I) having three monomer units.

The polyalkoxyamine and the acylating agent are suitably reacted in proportions such as to produce mainly either the mono- or the bis-succinimide derivative, or a mixture thereof.

Generally, the reaction will be effected in the presence of a solvent for the reactants. In view of the intended use of the product, a preferred solvent is a lubricating oil. The lubricating oil may be an animal, vegetable or mineral oil. Suitably the lubricating oil may be a petroleum-derived lubricating oil, such as a naphthenic base, paraffin base or mixed base oil. Solvent neutral oils are particularly suitable. Alternatively, the lubricating oil may be a synthetic lubricating oil. Suitable synthetic lubricating oils include synthetic ester lubricating oils, which oils include diesters such as di-octyl adipate, di-octyl sebacate and tri-decyladipate, or polymeric hydrocarbon lubricating oils, for example liquid polyisobutenes and poly-alpha olefins. Alternatively, the solvent may be an inert hydrocarbon, suitably a liquid paraffin. Generally, the dispersant additive will be transported as a concentrate in the solvent in which it is prepared.

The elevated temperature may suitably be in the range from 80 to 230, preferably from 120 to 210°C.

In another aspect the present invention provides a lubricating oil composition which composition comprises a major proportion of a lubricating oil and a minor proportion of a dispersant additive as hereinbefore described.

The lubricating oil may be any natural or synthetic lubricating oil. It may be any of the lubricating oils as described hereinbefore.

Into the lubricating oil composition there may also be incorporated any of the conventional additives normally employed, which additives include antioxidants, detergents, extreme pressure/anti-wear agents and viscosity index improvers. It is an advantage of the present invention that, because the dispersant composition of the invention has viscosity index properties, less of the conventional viscosity index improver may be required.

The lubricating oil composition may be used for any lubricating application, including automotive and marine use.

For automotive use the lubricating oil composition may suitably contain up to 10% (eg 0.1-10% such as 5-10%) by weight of the dispersant additive.

For marine engine use the lubricating oil composition may suitably contain up to 10% (eg 0.1-10% such as 5-10%) by weight of the dispersant additive.

In a final aspect the present invention provides a fuel composition comprising a major proportion of an internal combustion engine fuel and a minor proportion of a dispersant additive as described hereinbefore.

The fuel may suitably be either a mixture of liquid hydrocarbons boiling within the gasoline boiling range or a mixture of hydrocarbons boiling within the diesel boiling range.

Suitably the fuel composition may contain the additive in an amount up to 2%w/w (eg 0.01-2%) based on the total weight of the composition.

The fuel composition may contain additional additives commonly employed in fuels for example antioxidants, anti-knock additives, flame speed improvers and the like.

The invention will now be illustrated by reference to the following Examples.

## POLYALKOXYAMINE PRODUCTION

### Example 1

Polymerisation of triethyleneglycoldiamine to afford a polymer containing ca 6 monomer units

A one litre flanged flask was fitted with an overhead stirrer, condenser and nitrogen purge. The flask was then charged with 300g triethyleneglycoldiamine (2.03 mol) and 15g Raney nickel (5% w/w, water washed until

the wash water had a pH of 7). The flask was heated to 200°C with stirring and nitrogen purge for 230 minutes, then allowed to cool. The product (A) was dissolved in methanol (300 $cm^3$), the Raney nickel removed by filtration, and then the methanol solvent removed in vacuo. The product was a viscous red-brown liquid, yield 225g. Analysis of the $^{13}C$ nmr spectrum of the product (A) indicated a polymer containing ca 6 monomer units.

Example 2

Polymerisation of triethyleneglycoldiamine to afford a polymer containing ca 2,3, and 13 monomer units respectively

A one litre flanged flask was fitted with an overhead stirrer, condenser and nitrogen purge. The flask was then charged with 250g triethyleneglycoldiamine (1.69 mol) and 12.5g Raney nickel (5% w/w, water washed until the wash water had a pH of 7). The flask was heated to 160°C with stirring and nitrogen purge for 85 minutes, then the temperature was raised to 180°C. After a further 227 minutes a 50 $cm^3$ sample was taken [product (B)]. After a further 90 minutes at 180°C a second 50 $cm^3$ sample was taken [product (C)]. The reaction was heated for a further 200 minutes and then allowed to cool [product (D)]. The three products were dissolved individually in ethanol, the Raney nickel removed by filtration, and then the ethanol solvent removed in vacuo. The products are described below:

| Product | Appearance | Yield | Approximate number of monomer units by $^{13}$Cnmr |
|---|---|---|---|
| (B) | yellow liquid | 45g | 2 |
| (C) | orange viscous liquid | 40g | 3 |
| (D) | dark brown highly viscous liquid | 70g | 13 |

Example 3

Polymerisation of triethyleneglycoldiamine to afford a polymer containing ca3 monomer units

A one litre flanged flask was fitted with an overhead stirrer, condenser and nitrogen purge. The flask was then charged with 312g triethyleneglycoldiamine (2.11 mol) and 20g Raney nickel (6.7%w/w, water washed until the wash water had a pH of 7). The flask was heated to 175°C with stirring and nitrogen purge for 180 minutes, then allowed to cool. The product (E) was dissolved in methanol (300$cm^3$), the Raney nickel removed by filtration, and then the methanol solvent removed in vacuo. The product was a viscous red-brown liquid, yield 251g. Analysis of the $^{13}C$ nmr spectrum of the product (E) indicated a polymer containing ca3 monomer units.

Example 4

A 1 litre stainless steel autoclave was charged with triethyleneglycol (330g), liquid ammonia (72g(200 ml)) and a Raney nickel catalyst (30.27g). The vessel was sealed and pressurised with a further 10 barg (at ambient temperature) of hydrogen. The mixture was heated to 180°C and kept at that temperature for 12 hours. On cooling, the mixture was dissolved in methanol and filtered to remove the Raney nickel. Removal of the solvent gave a brown liquid which $^1H$ NMR showed to be a diamine of the formula (IX). The conversion of the triethyleneglycol to this product was 75%. The product was transferred to a glass reaction vessel and reacted in the same manner as for Example 3 to produce a polyalkoxyamine having the formula (I).

DISPERSANT PRODUCTION

In the following Examples reference will be made to '% actives'. The term 'actives' refers to any material that is not mineral oil.

Example 5

Polyisobutene succinic anhydride (PIBSA, 80g, wherein the polyisobutene substituent is derived from a mixture of polyisobutenes of Mn 1000 and 2400) and the product (C) of Example 2 (13.4g) were mixed, heated to 180°C, held at this temperature whilst stirring for 3hr, and stripped (10mm Hg) for 1 hr.

After cooling, a sample of the product was dissolved in 150SN oil to make a solution containing 7.3% actives of the component. Its' viscometric behaviour was examined and the results are given in Table 1.

Example 6

Example 5 was repeated except that PIBSA (630g) was reacted with the product (A) of Example 1 (142g).

A sample was dissolved in 150SN oil to make a solution containing 5.5% actives of the component, and the viscometric behaviour was examined. The material was also examined by a bench test indicative of dispersancy and by a Petter AVB (50 hour) engine test. The results are given in Table 1.

Example 7

Example 5 was repeated except that PIBSA (400g) was reacted with the product (C) of Example 2 (24.8g).

After cooling, a sample of the product was dissolved in 150SN oil to make a solution containing 7.3% actives of the component. Its' viscometric behaviour was examined and the results are given in Table 1.

The material was also examined by a Petter AVB (50 hour) engine test. The results are given in Table. 1.

Example 8

Example 5 was repeated except that PIBSA (800g) was reacted with the product (E) (28.3g) of Example 3.

After cooling, a sample of the product was dissolved in 150SN oil to make a solution containing 7.3% actives of the component. Its' viscometric behaviour was examined and the results are given in Table 1.

The product was also examined in a Petter AVB (50 hour) engine test (the results are given in Table 1) and in a VW seals swell test (VW10/98) (the results are given in Table 2).

It can be seen from the results presented in Table 2 that at the same 5% treat level the bis-succinimide derived from a polyalkoxyamine is better than the bis-succinimide derived from TEPA in the Seals Swell Test.

TABLE 1

| Product | Conc. actives | Vis 100°C cSt | Vis 40°C cSt | Vis -20°C P | VI | Dispersancy test | Petter AVB |
|---|---|---|---|---|---|---|---|
| Example 5 | 7.3% | 7.50 | 47.9 | 38 | 121 | - | - |
| Example 6 | 5.5% | 6.72 | 40.9 | 33 | 119 | Pass | Pass |
| Example 7 | 7.3% | 7.41 | 47.2 | - | 120 | - | Pass |
| Example 8 | 7.3% | 7.67 | 49.7 | 38 | 121 | - | Pass |

Table 2

| Product | Rubber 1 | | Rubber 2 | | Rubber 3 | |
|---|---|---|---|---|---|---|
| | TS | EB | TS | EB | TS | EB |
| A' | P | BL | P | BL | F | F |
| B' | P | F | P | BL | BL | BL |
| Product of Example 8 | BL | P | P | BL | P | P |

Product A' is a solution in 150SN oil of the bis-succinimide obtained by reacting tetraethylene pentamine (TEPA) with a polyisobutene succinic anhydride in which the polyisobutene substituent has an Mn of 1000 (73% actives).

Product B' is the bis-succinimide obtained by reacting TEPA with a polyisobutene succinic anhydride in which the polyisobutene is a 40:60 mixture of polyisobutenes of Mn 1000 and 2400 respectively (ie Mn is about 1840) (73% actives).

TS = Tensile Strength.

EB = Elongation at Break.

P = Pass; BL = Borderline; F = Fail

## Claims

1. A polyalkoxyamine of the formula:-

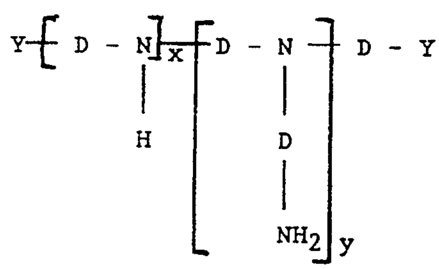

(I)

wherein (x+y) has a value in the range from 1 to 19 and y is either zero or an integer;

D is $\{CHR\text{-}CHR^1\text{-}O\}_n CHR^1\text{-}CHR\text{-}$ (II)

wherein in the formula (II) either $R=R^1=H$ or $R=C_1$ to $C_4$ alkyl and $R^1=H$ and n has a value in the range from 1 to 10; and

Y is independently either -OH or $-NHR^2$ wherein $R^2$ is either H or alkyl.

2. A polyalkoxyamine according to claim 1 wherein (x+y) has a value from 2 to 18; D is $\{CHR\text{-}CHR^1\text{-}O\}_n CHR^1\text{-}CHR$ wherein either $R=R_1=H$ or $R=CH_3$ and $R^1=H$ and n is either two or three; and Y is $-NH_2$.

3. A polyalkoxyamine according to either claim 1 or claim 2 wherein the value of (x+y) is less than 10.

4. A polyalkoxyamine according to claim 3 wherein the value of (x+y) is less than 5.

5. A polyalkoxyamine according to any one of the preceding claims wherein y is either one or zero.

6. A polyalkoxyamine according to any one of the preceding claims wherein Y is $-NH_2$.

7. A polyalkoxyamine according to claim 1 wherein in the formulae (I) and (II):-
$Y = -NH_2$;
$D = [CH_2-CH_2-O]_nCH_2-CH_2-$ wherein n is either 2 or 3;
(x+y) = either 2 or 3; and
y = either zero or one.

8. A mixture of two or more polyalkoxyamines having the formula (I).

9. A process for the production of a polyalkoxyamine having the formula (I), or a mixture thereof, which process comprises reacting at elevated temperature and in the presence of a transition metal catalyst a diamine having the formula:

$$R_2HN[CHR-CHR^1-O]_nCHR^1-CHR-NHR^2 \qquad (IX)$$

wherein in the formula (IX) R,$R^1$, $R^2$ and n have the same meaning as in the formulae (I) and (II).

10. A process according to claim 9 wherein $R^2$ in the formula (IX) is hydrogen.

11. A process according to either claim 9 or claim 10 wherein the diamine of the formula (IX) is triethyleneglycoldiamine.

12. A process according to any one of claims 9 to 11 wherein the catalyst is Raney nickel.

13. A process for the production of a polyalkoxyamine having the formula (I) which process comprises in a first step reacting ammonia at elevated temperature and in the presence of a transition metal catalyst and hydrogen with a glycol having the formula:-

$$HO[CHR-CHR^1-O]_nCHR^1-CHR-OH \qquad (X)$$

wherein in the formula (X) R,$R^1$ and n have the same meaning as in the formulae (I) and (II) to produce a diamine having the formula (IX) and in a second step reacting at elevated temperature and in the presence of a transition metal catalyst the diamine having the formula (IX).

14. A process according to claim 13 wherein the glycol is triethyleneglycol.

15. A process according to either claim 13 or claim 14 wherein the catalyst is Raney nickel.

16. A dispersant additive suitable for use in lubricating oil and fuel compositions which additive is obtainable by reacting at elevated temperature a polyalkoxyamine having the formula (I), or a mixture thereof, with a hydrocarbyl-substituted succinic acylating agent.

17. A dispersant additive according to claim 16 wherein the acylating agent has the formula:-

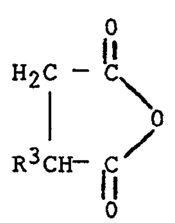

(XI)

wherein $R^3$ is an alkyl or alkenyl group derived from a polyolefin.

18. A dispersant additive according to claim 17 wherein the polyolefin is a polyisobutene.

19. A dispersant additive according to any one of claims 16 to 18 wherein the substituent $R^3$ in the formula (XI) has a number average molecular weight in the range from about 500 to about 5000.

**20.** A dispersant additive according to any one of claims 16 to 19 wherein there is reacted with the succinic acylating agent a mixture of polyalkoxyamines obtainable by the deaminative polymerisation of triethyleneglycoldiamine and containing a polyalkoxyamine of the formula (I) having three monomer units.

**21.** A dispersant additive according to any one of claims 16 to 20 in the form of a concentrate in the solvent in which it is prepared.

**22.** A lubricating oil composition comprising a major proportion of a lubricating oil and a minor proportion of a dispersant additive as claimed in claims 16 to 21.

**23.** A fuel composition comprising a major proportion of an internal combustion engine fuel and a minor proportion of a dispersant additive as claimed in claims 16 to 21.